**Europäisches Patentamt**

**European Patent Office**

**Office européen des brevets**

(19)

(11) Publication number : **0 447 267 B1**

(12) **EUROPEAN PATENT SPECIFICATION**

(45) Date of publication of patent specification :
01.06.94 Bulletin 94/22

(51) Int. Cl.⁵ : **B01J 23/50**, B01J 23/22, B01J 35/02, C07D 307/89

(21) Application number : 91302270.3

(22) Date of filing : 15.03.91

(54) **Catalyst for producing phthalic anhydride.**

(30) Priority : 16.03.90 JP 63975/90

(43) Date of publication of application :
18.09.91 Bulletin 91/38

(45) Publication of the grant of the patent :
01.06.94 Bulletin 94/22

(84) Designated Contracting States :
AT BE DE ES FR GB IT NL SE

(56) References cited :
EP-A- 17 865
EP-A- 37 492
Chemical Abstracts, vol 78, no.15, April 6, 1973, Columbus, Ohio, USA,SUZUKI HIDEO et al" Catalytic gas phase oxdation of naphthalene" page 455, column 1, abstrat, no 97 384z
Chemical abstract , vol 90, no 7, February 12, 79, Columbus ,Ohio, Usa, KRUSZKOWA, DANUTA et al:" Studies on selection of catalysts for partial oxidation of o-xylene" page 581, column 2, abstract no 54 624h
Chemical ABTRACTS, vol67, no 9, August 28, 1967, COlumbus, Ohio, US A,BADISCHE ANILIN- & SODAFABRIK AG" PURE PHTHALIC ANHYDIDE FROM O-XYLENE" Page 4082
Chemical abstracts, vol86, no 26, June 27,1977, Columbus, Ohio
CHEMICAL ABSTRACTS vol. 68, no. m25, June 17, 1968, Columbus, Ohio, USA, TADEUSZ WOJTOWICZ et al., page 11336, column 1, abstract no. 117

(73) Proprietor : NIPPON SHOKUBAI CO., LTD.
1-1, Koraibashi, 4-chome
Chuo-ku, Osaka-shi, Osaka (JP)

(72) Inventor : Ueda, Kenji, Nippon Shokubai Hamadaryo
931-11 Hamada,
Aboshi-ku
Himeji-shi, Hyogo (JP)
Inventor : Kawabata, Tatsuya
Ohtsu-Apart 69-22, 3-29, Ohtsu-cho, Ohtsu-ku
Himeji-shi, Hyogo (JP)
Inventor : Okuno, Masaaki, Nippon Shokubai Hamadaryo
931-11 Hamada,
Aboshi-ku
Himeji-shi, Hyogo (JP)
Inventor : Nishio, Chisako
Room 507,
942-26, Mutsuura-cho,
Kanazawa-ku
Yokohama-shi, Kanagawa (JP)
Inventor : Tanaka, Shinya
13-16, Taderahigashi 4-chome
Himeji-shi, Hyogo (JP)

(74) Representative : Geering, Keith Edwin et al
REDDIE & GROSE
16 Theobalds Road
London WC1X 8PL (GB)

**Description**

The present invention relates to a catalyst for producing phthalic anhydride, and more particularly to a catalyst for producing phthalic anhydride by vapor phase catalytic oxidation of o-xylene and/or naphthalene with molecular oxygen or gas containing molecular oxygen.

Catalysts for producing phthalic anhydride having a catalytic active substance mainly composed of vanadium oxide and titanium oxide supported on an inert carrier are widely known, and are disclosed, for example, in Japanese Patent Publications 47-15323, 49-41036, and Japanese Laid-open Patents 47-5661, 49-89694. These catalysts have their own features, and some of them are used industrially and achieving due results.

There is, however, ample room for improvement of catalyst performance - regarding selectivity an increase in yield of only 1% is notable in its economic effect considering the scale of production. Furthermore, improvement of selectivity facilitates heat treatment and distillation to obtain the product, and helps to produce products of high quality at low cost. Such improvement of selectivity is also important for using the raw materials effectively.

What is also important is improvement of productivity and maintenance of stable production by retaining catalyst activity. One method for enhancing productivity is to perform the oxidation under high load reaction conditions, e.g. raising the reactant gas concentration. However, production of phthalic anhydride from o-xylene or naphthalene is accompanied by violent heat generation, and the temperature rise in the hot spot area under high concentration conditions is extreme, so that excessive oxidation occurs, the yield of phthalic anhydride is lowered, and deterioration of the catalyst is extreme.

Catalysts capable of withstanding such high load reaction conditions have been already proposed, for example, by the present applicant in Japanese Patent Publication 59-1378 and EP-A 17 865.

We have found that introducing silver into the vanadium-titanium catalyst gives improvement.

The invention provides a catalyst for producing phthalic anhydride by vapor phase catalytic oxidation of o-xylene and/or naphthalene with molecular oxygen or gas containing molecular oxygen, having supported on a heat resistant inorganic carrier catalytic active substance comprising (A) 1 to 20 parts by weight of vanadium oxide as $V_2O_5$ and 99 to 80 parts by weight of anatase type titanium dioxide with specific surface area of 10 to 60 $m^2/g$ as $TiO_2$ and (B), per 100 parts by total weight of (A), 0.05 to 1.2 parts by weight of at least one element selected from potassium, cesium, rubidium and thallium as oxide, and 0.05 to 2 parts by weight of silver as $Ag_2O$ ; (hereinafter this is called catalyst 1).

The invention moreover provides a catalyst for producing phthalic anhydride by vapor phase catalytic oxidation of o-xylene and/or naphthalene with molecular oxygen or gas containing molecular oxygen, having supported on a heat resistant inorganic carrier a catalytic active substance comprising (A) 1 to 20 parts by weight of vanadium as $V_2O_5$ and 99 to 80 parts by weight of anatase type titanium dioxide with specific surface area of 10 to 60 $m^2/g$ as $TiO_2$ and (C), per 100 parts by total weight of (A), 0 to 1 part by weight of niobium as $Nb_2O_5$, 0.05 to 1.2 parts by weight of at least one element selected from potassium, cesium, rubidium and thallium as oxide, 0 to 1.2 parts by weight of phosphorus as $P_2O_5$, 0 to 5 parts by weight of antimony as $Sb_2O_3$, and 0.05 to 2 parts by weight of silver as $Ag_2O$, the total niobium, phosphorus and antimony content not being zero; hereinafter this is called catalyst 2.

It is one of the features of the invention that anatase type titanium dioxide with specific surface area of 10 to 60 $m^2/g$, preferably 15 to 40 $m^2/g$, is used as a component of the catalytic active substance.

If the specific surface area of the anatase type titanium dioxide is less than 10 $m^2/g$, the activity of the obtained catalyst is low ; when it exceeds 60 $m^2/g$, the durability of the catalyst is reduced, and the yield rapidly decreases, which is not preferable.

Anatase type titanium dioxide with a mean particle size of 0.4 to 0.7 $\mu m$, preferably 0.45 to 0.60 $\mu m$, and of substantially spherical form is favoured for use.

An anatase type titanium dioxide preferred for in the invention is manufactured by a method called the sulfuric acid solution method, and is high in mechanical strength in spite of porosity; it possesses such strength as to be regarded as "primary particles" not crushed by mechanical grinding by an ordinary ball mill or the like. Although this anatase type titanium oxide has a large mean particle size of 0.4 to 0.7 $\mu m$, it has a high specific surface area of 10 to 60 $m^2/g$, and it is essentially an assembly of primary particles of small diameter. Therefore, this anatase type titanium dioxide is not required to be true spheres, but suffices if it is substantially spherical.

According to the sulfuric acid solution method, ilmenite ($FeOTiO_2$) is treated with sulfuric acid at a lower concentration than in production of titanium dioxide by the sulfuric acid solidification method, usually with sulfuric acid at about 70 to 80%, to obtain titanium sulfate, and this titanium sulfate is hydrolyzed under pressure at 150 to 180°C, and is further baked at 600 to 900°C to obtain anatase type titanium oxide. This anatase type titanium oxide may contain (from the ores) iron, zinc, aluminum, manganese, chromium, calcium or lead as oxides, but from the viewpoint of catalytic performance it does not matter particularly if such oxide contents in titanium oxide are not more than 0.5 wt.%.

The heat resistant inorganic carrier used in the invention is required to be stable for a long period at a temperature higher than the catalyst operating and the catalyst baking temperatures and not to react with the catalytic active substance.

Examples of such heat resistant inorganic carrier include, among others, silicon carbide (SiC), alumina, zirconium oxide, and titanium oxide. Particularly preferable among them is a silicon carbide carrier with an alumina ($Al_2O_3$) content of 20 wt.% or less, preferably 5 wt.% or less, and apparent porosity of 10% or more, preferably 15 to 45%. In particular, a silicon carbide carrier with an alumina content of 5 wt.% or less, silicon carbide content of 95 wt.% or more, and apparent porosity of 15 to 45% is favorably used. A silicon carbide carrier obtained by self-sintering of silicon carbide powder with a purity of 98% or more is preferred.

The shape of the heat resistant inorganic carrier is not particularly limited, but spherical or columnar shapes are easy to handle, and a mean diameter of about 2 to 15 mm is preferred.

Catalyst 1 of the invention is obtainable by supporting, on heat resistant inorganic carrier, a catalytic active ingredient comprising 1 to 20 parts by weight of vanadium oxide as $V_2O_5$, 99 to 80 parts by weight of anatase type titanium oxide as $TiO_2$ and, per 100 parts by weight of the sum of these two ingredients, 0.05 to 1.2 parts by weight of at least one element selected from potassium, cesium, rubidium and thallium as oxide, and 0.05 to 2 parts by weight of silver as $Ag_2O$.

It is one of the features of the invention, as mentioned hereabove, that silver is introduced as an ingredient of the catalytic active substance, and the silver content in catalyst 1 is to 0.05 to 2 (preferably 0.1 to 1) parts by weight as $Ag_2O$ per 100 parts by weight of (A). The silver content should not be too high or too low. If the silver content as $Ag_2O$ is less than 0.05 part by weight, the effect of improving performance by addition of silver is lowered. If it exceeds 2 parts by weight, adverse effects are brought about on catalyst performance, and the selectivity to phthalic anhydride is lowered.

Catalyst 2 of the invention is obtainable by supporting, on heat resistant inorganic carrier, a catalyst active ingredient comprising 1 to 20 parts by weight of vanadium oxide as $V_2O_5$, 99 to 80 parts by weight of anatase type titanium oxide as $TiO_2$ and, per 100 parts by weight of the total of these two ingredients, 0 to 1 part by weight of niobium as $Nb_2O_5$, 0.05 to 1.2 parts by weight of at least one element selected from potassium, cesium, rubidium and thallium as oxide, 0 to 1.2 parts by weight of phosphorus as $P_2O_5$, 0 to 5 parts by weight of antimony as $Sb_2O_3$, and 0.05 to 2 parts by weight of silver as $Ag_2O$ (where contents of niobium, phosphorus and antimony will not be zero at the same time).

In catalyst 2, as in catalyst 1, the Ag content as $Ag_2O$ is 0.5 to 2 (preferably 0.1 to 1) parts by weight per 100 parts by weight of (A). The silver content should not be too high or too low.

When catalyst 2 contains 0.01 to 1 part by weight of niobium as $Nb_2O_5$, 0.2 to 1.2 parts by weight of phosphorus as $P_2O_5$, and 0.5 to 5 parts by weight of antimony as $Sb_2O_3$, the selectivity to phthalic anhydride is enhanced.

The vanadium, niobium, potassium, cesium, rubidium, thallium, phosphorus and antimony starting materials for preparing catalysts 1 and 2 may be selected from, aside from such oxides as $V_2O_5$, $Nb_2O_5$, $K_2O$, $Cs_2O$, $Rb_2O$, $Tl_2O$, $P_2O_5$ and $Sb_2O_3$, compounds transformed to such oxide by heating - such as ammonium salts, nitrates, sulfates, halides, organic acid salts and hydroxides of the individual elements.

As for the silver ingredient, aside from $Ag_2O$, a nitrate, ammonium salt, sulfate, halide, organic acid salt, hydroxide, amine complex, phosphate or sulfide may be used. Some of them, such as silver halide and silver phosphate, are not transformed into oxide by heating in the production of the catalyst, but all of them may be used in the invention without trouble. Besides, when silver phosphate is used, or when adding phosphorus ingredient as catalytic active substance, it is not necessary to consider the content of phosphorus in the silver phosphate, and it is all right as far as the content as the oxide of phosphorus ingredient is within the prescribed range.

The method of supporting the catalytic active substance on the heat resistant inorganic carrier in production of catalysts of the invention is not particularly limited, and various known methods can be used. A simple method is to put a specific volume of carrier into a rotary drum which can be heated from outside, and spray it with a slurry of the catalytic active substance while keeping it at 200 to 300°C.

The amount of catalytic active substance on the heat resistant inorganic carrier varies with the carrier size, and it is usually to 3 to 20 g per 100 cc of the carrier.

The catalytic active substance layer obtained by supporting the catalytic active substance on the carrier preferably has a surface such that 50% or more (preferably 75% or more) of the total fine pore volume (that occupied with a diameter of 10 $\mu$m or less) is occupied by pores of 0.15 to 0.45 $\mu$m diameter Such a catalytic layer be easily formed by adjusting the slurry concentration according to the particle size of the essential primary particles of the anatase type titanium oxide, in the supporting method using, for example, a rotary drum as mentioned above (see Japanese Patent Publication 49-41036). For example when using anatase type titanium oxide with a primary particle size of 0.005 to 0.05 µm, the slurry concentration may be 5 to 25 wt.%, preferably 10 to 20 wt.%; and when using anatase type titanium dioxide with a primary particle size of 0.05 µm or greater, the slurry concentration may be 10 to 40 wt.%, preferably 15 to 25 wt.%,

Herein, the fine pore volume is determined from the fine pore diameter distribution measured by mercury injection porosimeter. The specific surface area of anatase type titanium dioxide is measured by the BET method, and the mean diameter is measured using a transmission electron microscope.

After thus supporting the catalytic active substance layer, the catalyst of the invention can be obtained by baking in passing air for 2 to 10 hours at a temperature of 450 to 700°C, preferably 500 to 600°C.

The oxidation of o-xylene and/or naphthalene using the catalyst of the invention may be executed under usual reaction conditions. For example, a reaction tube of inside diameter 5 to 40 mm, preferably 15 to 27 mm, is filled with the catalyst to a height of 1 to 5 m, preferably 1.5 to 3 m, and held at a temperature of 300 to 400°C, preferably 330 to 380°C; into this reaction tube the o-xylene and/or naphthalene is blown, together with air or gas containing 5 to 21 vol.% molecular oxygen, at a rate of 5 to 70 g/Nm$^3$ (air) in the case of air, or 5 to 110 g/Nm$^3$ (gas containing molecular oxygen) in the case of gas containing molecular oxygen, at a spatial velocity of 1,000 to 6,000 hr$^{-1}$ (STP), preferably 1,000 to 4,000 hr$^{-1}$ (STP).

It can be advantageous to divide the catalyst in the reaction tube into two or more layers to give a plurality of reaction bands, with a plurality of catalysts of controlled activity disposed in these bands so that the activity is higher from the gas inlet towards the outlet.

For example, when using catalyst 2 the reaction tube may be divided into two layers, and the inlet part filled with specified catalyst (first stage catalyst) to a layer height of 30 to 70% of the overall catalyst layer height while the remaining layer height at the outlet is filled with catalyst (second stage catalyst) of higher activity than the first stage catalyst. Catalysts of the same type but different in activity may be easily prepared by changing the content of, for example, phosphorus. Thus using 0.2 to 0.4 part by weight of phosphorus as oxide per 100 parts by weight of (A), an above first stage catalyst may be prepared, and using 0.4 to 1.2 parts by weight a second stage catalyst of higher activity may be prepared. Catalyst activity may also be controlled by changing the type and/or content of element selected from potassium, cesium, rubidium and thallium.

By performing the reaction under such conditions as mentioned above, heat accumulation in the hot spot in the catalyst layer is suppressed, and catalyst deterioration due to thermal load is reduced, so that stable long term industrial operation is possible.

Excessive oxidation reaction due to the hot spot is prevented, and various effects are obtained, including improvement of selectivity. Such effects are particularly notable under high load reaction conditions such as high gas flow, and the productivity may be markedly enhanced by raising the concentration of o-xylene or naphthalene.

By using the catalysts of the invention, phthalic anhydride may be manufactured at high selectivity from o-xylene and/or naphthalene. The operations of heat treatment and distillation to obtain phthalic anhydride product are facilitated and products of higher quality may be obtained at lower cost as compared with conventional methods.

The catalysts of the invention are durable, and accordingly long-term stable operation is possible industrially.

The invention is illustrated by the following Examples of some preferred embodiments in comparison with Reference Examples not according to the invention.

Example 1

(Preparation of catalyst)

After mixing 80% concentrated sulfuric acid with ilmenite and reacting, an aqueous solution of titanium sulfate was obtained by diluting in water. Iron pieces were added to this as reducing agent, and the iron content from the ilmenite was reduced to ferrous ions; after cooling ferrous sulfate was precipitated and separated. Into the obtained aqueous solution of titanium sulfate, steam heated to 150°C was blown, and hydrous titanium oxide settled. It was washed in water, pickled and washed again in water, and was baked at 800°C for 4 hours in a passing air flow. It was crushed by jet air stream to obtain anatase type titanium oxide (hereinafter sometimes called simply titanium dioxide) with a mean particle size of about 0.5 µm and specific surface area of 22 m$^2$/g.

In 6,400 ml of deionized water, 200 g of oxalic acid was dissolved, and 47.25 g of ammonium metavanadate, 5.98 g of ammonium dihydrogen phosphate, 18.79 g of niobium chloride, 5.90 g of cesium sulfate, 5.39 g of silver nitrate, and 36.73 g of antimony trioxide were added and stirred. To the obtained solution, 1,800 g of titanium dioxide was added, and was stirred by emulsifying machine to prepare a slurry.

In a stainless steel rotary furnace of 35 cm diameter and 80 cm length which can be heated from outside, 2,000 ml of SiC self-sintered carrier of spherical form with diameter of 6 mm and apparent porosity of 35% was charged, and while rotating the furnace preheated to 200 to 250°C, the slurry was sprayed onto the carrier; the catalytic active substance was thus supported at a rate of 8 g/100 ml (carrier). Afterwards, in passing air, it was baked in an electric oven for 6 hours at 580°C, to prepare catalyst A.

Table 1 shows the composition of catalyst A, the ratio in the catalytic active substance layer (vol.%) of the pore volume occupied by pores of diameter 0.15 to 0.45 μm to the total fine pore volume occupied by pores of 10 μm or less diameter, and the specific surface area and mean particle size of the titanium dioxide used in preparation of the catalyst (these are collectively) called catalyst characteristics hereinafter). The said pore volume ratio is determined from the fine pore distribution measured by the Hg injection porosimeter.

Catalyst B was prepared as for catalyst A, except that the content of ammonium dihydrogen phosphate was 23.92 g.

The catalyst characteristics of catalyst B are shown in Table 1.

The phosphorus content in catalyst B was higher than that in catalyst A, and the activity of catalyst B was higher than that of catalyst A.

(Oxidation Reaction)

Into an iron reaction tube of 25 mm inside diameter and 3 m length immersed in a molten salt bath kept at 355°C, catalyst B was charged as second stage catalyst to a height of 1 m at the gas outlet end, then catalyst A as first stage catalyst was charged to a height of 1.5 m at the inlet.

O-xylene was mixed at a ratio of 85 g/Nm³ (synthetic gas) with synthetic gas comprising 10 vol.% of oxygen, 10 vol.% of steam and 80 vol.% of nitrogen, and this mixture was led into the upper inlet of the reaction tube at a space velocity (SV) of 2,500 hr⁻¹ (STP) to oxidize o-xylene.

At the beginning of reaction, 3 months after start of reaction and 6 months after start of reaction, the yield of phthalic anhydride was measured, and the results are shown in Table 2. The conversion rate of orthoxylene is nearly 100%, and this yield can be regarded as the selectivity to phthalic anhydride.

Example 2

Catalyst C and catalyst D were prepared as in Example 1 except that 4.94 g of silver sulfate was used instead of 5.39 g of silver nitrate; the oxidation reaction was conducted as in Example 1.

The catalyst characteristics of catalysts C, D are shown in Table 1, and results of oxidation reaction in Table 2.

Example 3

Catalyst E and catalyst F were prepared as in Example 1, except that 4.42 g of silver phosphate was used instead of 5.39 g of silver nitrate; the oxidation reaction was conducted as in Example 1.

The catalyst characteristics of catalysts E, F are shown in Table 1, and results of oxidation reaction in Table 2.

Reference Example 1

Catalysts K, L were prepared as in Example 1, except that the content of cesium sulfate was 8.25 g and that silver was not added; the oxidation reaction was conducted as in Example 1.

The catalyst characteristics of catalysts K, L are shown in Table 1, and results of oxidation reaction in Table 2.

Example 4

(Preparation of catalyst)

Ilmenite and 80% concentrated sulfuric acid were mixed and allowed to react, then the product was diluted to obtain an aqueous solution of titanium sulfate. Iron pieces were added as reducing agent, and the iron from the ilmenite was reduced to ferrous ions; after cooling ferrous sulfate was precipitated and separated. Into the obtained aqueous solution of titanium sulfate, steam heated to 150°C was blown, and hydrous titanium oxide settled. It was washed in water, pickled, and washed again in water, and was baked at 700°C for 4 hours in a passing air flow. It was crushed by jet air stream, and anatase type titanium dioxide with specific surface area

of 33 m$^2$/g and mean particle size of about 0.45 μm was obtained.

In 6,400 ml of deionized water, 900 g of oxalic acid was dissolved, and to this aqueous solution 408.60 g ammonium metavanadate, 10.34 g of ammonium dihydrogen phosphate, 17.33 g of niobium chloride, 2.72 g of cesium sulfate, 3.92 g of potassium sulfate, 31.05 g of silver nitrate, and 42.35 g of antimony trioxide were added and stirred. To the obtained solution, 1,800 g of titanium dioxide was added, and the mixture was stirred by emulsifying machine to prepare a catalyst slurry.

Using this slurry, the catalytic active substance was supported as in Example 1 at a supporting rate pf 8.0 g/100 ml of carrier, followed by baking in passing air in an electric oven at 560°C for 6 hours to prepare catalyst G.

Catalyst H was prepared as for catalyst G, except that the content of ammonium dihydrogen phosphate was 31.02 g.

(Oxidation Reaction)

Into an iron reaction tube of 25 mm inside diameter and 3 m length immersed in a molten salt bath kept at 365°C, catalyst H was charged as second stage catalyst to a height of 1 m, then catalyst G as first stage catalyst to a height of 1.5 m; to the upper part of the reaction tube naphthalene, mixed at a ratio of 85 g/Nm$^3$ (synthetic gas) with synthetic gas comprising 10 vol.% of oxygen, 10 vol.% of steam and 80 vol.% of nitrogen, was introduced at a space velocity of 2,500 hr$^{-1}$ (STP) to perform the oxidation.

The catalyst characteristics of catalysts G, H are shown in Table 1, and results of oxidation reaction in Table 2.

Reference Example 2

Catalysts M, N were prepared as for catalysts G, H in Example 4, except that the content of potassium sulfate was 1.96 and the content of silver nitrate was 77.63 g; the oxidation reaction was performed as in Example 4.

The catalyst characteristics of catalysts M, N are shown in Table 1, and results of oxidation reaction in Table 2.

Example 5

(Preparation of Catalyst)

In 6,400 cc of deionized water, 200 g of oxalic acid was dissolved, and to this aqueous solution 96.48 g of ammonium metavanadate, 4.82 g of cesium sulfate, 1.18 g of thallium nitrate and 2.75 g of silver nitrate were added and stirred.

To the obtained solution, the same anatase titanium dioxide as used in Example 1 was added (1,800 g as TiO$_2$) and the mixture was stirred by emulsifying machine to obtain a slurry.

Using the slurry, the catalytic active substance was supported in the same manner as in Example 1 at a support rate of 8.0 g/100 ml of carrier followed by baking in passing air in an electric oven at 550°C for 6 hours to obtain catalyst I (first stage catalyst).

Catalyst J (second stage catalyst) was prepared as for catalyst I, except that 2.96 g of rubidium nitrate was used instead of cesium sulfate and thallium nitrate.

(Oxidation Reaction)

The reaction was conducted as in Example 1, except that a mixture of 70 g/Nm$^3$ (synthetic gas) of orthoxylene with synthetic gas comprising 21 vol.% of oxygen and 79 vol.% of nitrogen was used and that this mixed gas was introduced from the upper inlet of the reaction tube at a space velocity of 3,000 hr$^{-1}$ (STP).

The catalyst characteristics of catalysts I, J are shown in Table 1, and results of oxidation reaction in Table 2.

Example 6

Catalyst Q was prepared as in Example 5 for preparation of catalyst I, except that 19.06 g of niobium chloride was added.

Catalyst R was prepared as for catalyst J, except that the content of rubidium nitrate was 4.44 g and that 6.08 g of ammonium dihydrogen phosphate was added.

The oxidation reaction was conducted as in Example 5.

The catalyst characteristics of catalysts Q, R are shown in Table 1, and the results of oxidation reaction in Table 2.

Example 7

Catalysts S, T were prepared as in Example 5, except that 18.75 g of antimony trioxide was added, and the oxidation reaction was conducted as in Example 5.

The catalyst characteristics of catalysts S, T are shown in Table 1, and the results of oxidation reaction in Table 2.

Example 8

Catalyst U was prepared as in Example 5 for catalyst I, except that the content of cesium sulfate was 6.02 g, and that 19.06 g of niobium chloride and 6.08 g of ammonium dihydrogen phosphate were added.

Catalyst V was prepared as for catalyst J, except that the content of rubidium nitrate was 4.44 g, and that 6.08 g of ammonium dihydrogen phosphate and 18.75 g of antimony trioxide were added.

The oxidation reaction was conducted as in Example 5.

The catalyst characteristics of catalysts U, V are shown in Table 1, and the results of oxidation reaction in Table 2.

Example 9

Catalyst W was prepared as in Example 5 for catalyst I, except that 19.06 g of niobium chloride and 18.75 g of antimony trioxide were added.

Catalyst X was prepared as for catalyst J, except that the content of rubidium nitrate was 4.44 g, and that 19.06 g of niobium chloride, 6.08 g of ammonium dihydrogen phosphate, and 18.75 g of antimony trioxide were added.

The oxidation reaction was conducted as in Example 5.

The catalyst characteristics of catalysts W, X are shown in Table 1, and the results of oxidation reaction in Table 2.

Reference Example 3

Catalyst O (first stage catalyst) was prepared as in Example 5 for catalyst I, except that the content of cesium sulfate was 6.03 g and that silver nitrate was not added.

Catalyst P (second stage catalyst) was prepared as for O, except that the content of rubidium nitrate was 4.44 g and that silver nitrate was not added.

Thereafter, the oxidation reaction was conducted as in Example 5.

The catalyst characteristics of catalysts O, P are shown in Table 1, and the results of oxidation reaction in Table 2.

In these foregoing examples and reference examples the oxidation reaction was conducted with constant feed to the catalyst; in the case of orthoxylene the molten salt temperature was set so that the by-product phthalide was controlled to under 0.1 wt.%, while in the case of naphthalene, it was set so that the by-product naphthaquinone could be controlled to under 0.5 wt.%.

Table 1

| | Type of catalyst | Catalyst composition (ratio by weight) | | | | | | | | Titanium dioxide | | Pore volume ratio (*) vol.% |
|---|---|---|---|---|---|---|---|---|---|---|---|---|
| | | $V_2O_5$ | $TiO_2$ | $Nb_2O_5$ | $P_2O_5$ | $Cs_2O$ | $Ag_2O$ | $Sb_2O_3$ | $K_2O$ | Mean particle size μm | Specific surface area $m^2/g$ | |
| Example 1 | A | 2 | 98 | 0.5 | 0.2 | 0.25 | 0.2 | 2.0 | – | 0.5 | 22 | 86 |
| | B | 2 | 98 | 0.5 | 0.8 | 0.25 | 0.2 | 2.0 | – | 0.5 | 22 | 87 |
| Example 2 | C | 2 | 98 | 0.5 | 0.2 | 0.25 | 0.2 | 2.0 | – | 0.5 | 22 | 86 |
| | D | 2 | 98 | 0.5 | 0.8 | 0.25 | 0.2 | 2.0 | – | 0.5 | 22 | 87 |
| Example 3 | E | 2 | 98 | 0.5 | 0.2 | 0.25 | 0.2 | 2.0 | – | 0.5 | 22 | 86 |
| | F | 2 | 98 | 0.5 | 0.8 | 0.25 | 0.2 | 2.0 | – | 0.5 | 22 | 87 |
| Reference Example 1 | K | 2 | 98 | 0.5 | 0.2 | 0.35 | – | 2.0 | – | 0.5 | 22 | 86 |
| | L | 2 | 98 | 0.5 | 0.8 | 0.35 | – | 2.0 | – | 0.5 | 22 | 87 |
| Example 4 | G | 15 | 85 | 0.4 | 0.3 | 0.1 | 1.0 | 2.0 | 0.1 | 0.45 | 33 | 80 |
| | H | 15 | 85 | 0.4 | 0.9 | 0.1 | 1.0 | 2.0 | 0.1 | 0.45 | 33 | 81 |
| Reference Example 2 | M | 15 | 85 | 0.4 | 0.3 | 0.1 | 2.5 | 2.0 | 0.05 | 0.45 | 33 | 80 |
| | N | 15 | 85 | 0.4 | 0.9 | 0.1 | 2.5 | 2.0 | 0.05 | 0.45 | 33 | 81 |

EP 0 447 267 B1

## Table 1 (Continued)

| | Catalyst | Catalyst composition (ratio by weight) $V_2O_5:TiO_2:Nb_2O_5:P_2O_5:Cs_2O:Ag_2O:Sb_2O_3:Rb_2O:Tl_2O$ | Titanium dioxide | Pore volume ratio (*) vol. % |
|---|---|---|---|---|
| Example 5 | I | 4 : 96 : − : − :0.2 : 0.1: − : − :0.05 | mean particle size 0.5 μm | 85% |
| | J | 4 : 96 : − : − :− : 0.1: − : 0.1 :− | | 86% |
| Example 6 | Q | 4 : 96 : 0.5 : − :0.2 : 0.1: − : − :0.05 | | 85% |
| | R | 4 : 96 : − : 0.2:− : 0.1: − : 0.15: − | | 86% |
| Example 7 | S | 4 : 96 : − : − :0.2 : 0.1: 1.0 : − :0.05 | | 85% |
| | T | 4 : 96 : − : − :− : 0.1: 1.0 : 0.1 : − | | 86% |
| Example 8 | U | 4 : 96 : 0.5 : 0.2:0.25: 0.1: − : − :0.05 | specific surface area 22 $m^2/g$ | 85% |
| | V | 4 : 96 : − : 0.2: − : 0.1: 1.0 : 0.15: − | | 86% |
| Example 9 | W | 4 : 96 : 0.5 : − :0.2 : 0.1: 1.0 : − :0.05 | | 85% |
| | X | 4 : 96 : 0.5 : 0.2: − : 0.1: 1.0 : 0.15: − | | 86% |
| Reference Example 3 | O | 4 : 96 : − : − :0.25: − : − : − :0.05 | | 85% |
| | P | 4 : 96 : − : − : − : − : − : 0.15: − | | 86% |

\* ratio of pore volume occupied by pores of diameter of 0.15 to 0.45 μm to total fine pore volume occupied by pores of diameter of 10 μm or less.

EP 0 447 267 B1

Table 2

| | Type of catalyst | | Yield of phthalic anhydride (wt.%) | | |
|---|---|---|---|---|---|
| | first stage | second stage | Initial(*) | 3 months(*) | 6 months(*) |
| Example 1 | A | B | 114.8 (353) | 115.0 (349) | 115.0 (350) |
| Example 2 | C | D | 114.7 (353) | 115.0 (349) | 115.1 (350) |
| Example 3 | E | F | 114.9 (353) | 115.1 (349) | 115.1 (349) |
| Ref.Ex.1 | K | L | 112.5 (353) | 113.0 (349) | 113.0 (350) |
| Example 4 | G | H | 104.5 (363) | 104.7 (358) | 104.8 (357) |
| Ref.Ex.2 | M | N | 100.8 (365) | - | - |
| Example 5 | I | J | 113.5 (357) | 113.8 (354) | 113.8 (354) |
| Example 6 | Q | R | 113.6 (356) | 114.0 (352) | 114.1 (351) |
| Example 7 | S | T | 113.8 (358) | 113.9 (355) | 114.0 (354) |
| Example 8 | U | V | 113.8 (358) | 114.2 (353) | 114.0 (353) |
| Example 9 | W | X | 114.0 (355) | 114.3 (352) | 114.1 (352) |
| Ref.Ex.3 | O | P | 110.0 (360) | 110.5 (358) | 110.4 (357) |

```
(*) Molten salt temperature
    Examples 1 to 3, Ref.Ex.1   : orthoxylene to phthalic anhydride
    Example 4, Ref.Ex.2         : naphthalene to phthalic anhydride
    Example 5, Ref.Ex.3         : orthoxylene to phthalic anhydride
```

From comparison between Examples 1 to 3 and Reference Example 1, and between Examples 5 to 9 and Reference Example 3. the yield of phthalic anhydride is evidently improved by adding silver, and comparison between Example 4 and Reference Example 2 shows that there is limitation to the addition of silver.

As shown in Tables 1 and 2, the catalysts of the invention containing silver improved the yield of phthalic anhydride by about 2% as compared with the catalysts without silver, and their performance after three months and six months indicated great stability, giving great economic improvement. For example, taking a present production of phthalic anhydride of 40,000 tons a year, a yield increase of 2% gives an additional 400 tons of phthalic anhydride without increasing the consumption of materials.

## Claims

1. A catalyst for producing phthalic anhydride by vapor phase catalytic oxidation of o-xylene and/or naphthalene with molecular oxygen or gas containing molecular oxygen, having supported on a heat resistant inorganic carrier a catalytic active substance comprising

   (A) 1 to 20 parts by weight of vanadium oxide as $V_2O_5$ and 99 to 80 parts by weight of anatase type titanium dioxide with specific surface area of 10 to 60 m²/g as $TiO_2$ and (B), per 100 parts by total weight of (A), 0.05 to 1.2 parts by weight of at least one element selected from potassium, cesium, rubidium and thallium as oxide and 0.05 to 2 parts by weight of silver as $Ag_2O$ .

2. A catalyst for producing phthalic anhydride by vapor phase catalytic oxidation of o-xylene and/or naphthalene with molecular oxygen or gas containing molecular oxygen, having supported on a heat resistant inorganic carrier a catalytic active substance comprising (A) 1 to 20 parts by weight of vanadium as $V_2O_5$, and 99 to 80 parts by weight of anatase type titanium dioxide with specific surface area of 10 to 60 $m^2/g$ as $TiO_2$ and (C), per 100 parts by weight of (A), O to 1 part by weight of niobium as $Nb_2O_5$, 0.05 to 1.2 parts by weight of at least one element selected from potassium, cesium, rubidium and thallium as oxide, 0 to 1.2 parts by weight of phosphorus as $P_2O_5$, 0 to 5 parts by weight of antimony as $Sb_2O_3$, and 0.05 to 2 parts by weight of silver as $Ag_2O$ , the total content of niobium, phosphorus and antimony not being zero.

3. A catalyst according to claim 1 or 2 wherein, in the catalytic active substance layer supported on the heat resistant inorganic carrier, the pore volume occupied by pores of 0.15 to 0.45 $\mu$m diameter is 50% or more of the total fine pore volume occupied by fine pores of 10 $\mu$m or less diameter.

**Patentansprüche**

1. Katalysator zur Herstellung von Phthalsäureanhydrid durch katalytische Dampfphasen-Oxidation von o-Xylol und/oder Naphthalin mit molekularem Sauerstoff oder einem molekularen Sauerstoff enthaltenden Gas, der auf einem hitzebeständigen anorganischen Träger eine katalytisch aktive Substanz aufweist, enthaltend

   (A) 1 bis 20 Gew.-Teile Vanadiumoxid als $V_2O_5$ und 99 bis 80 Gew.-Teile Titanoxid vom Anatas-Typ mit einem spezifischen Oberflächenbereich von 10 bis 60 $m^2/g$ als $TiO_2$ und
   (B) je 100 Teile des Gesamtgewichtes von (A) 0,05 bis 1,2 Gew.-Teile mindestens eines Elements ausgewählt von Kalium, Caesium, Rubidium und Thallium als Oxide und 0,05 bis 2 Gew.-Teile Silber als $Ag_2O$.

2. Katalysator zur Herstellung von Phthalsäureanhydrid durch katalytische Dampfphasen-Oxidation von o-Xylol und/oder Naphthalin mit molekularem Sauerstoff oder einem molekularen Sauerstoff enthaltenden Gas, der auf einem hitzebeständigen anorganischen Träger eine katalytisch aktive Substanz aufweist, enthaltend

   (A) 1 bis 20 Gew.-Teile Vanadiumoxid als $V_2O_5$ und 99 bis 80 Gew.-Teile Titanoxid vom Anatas-Typ mit einem spezifischen Oberflächenbereich von 10 bis 60 $m^2/g$ als $TiO_2$ und
   (C) je 100 Teile des Gewichtes von (A) 0 bis 1 Gew.-Teil Niob als $Nb_2O_5$, o,05 bis 1,2 Gew.-Teile mindestens eines Elements von Kalium, Caesium, Rubidium und Thallium als Oxid, 0 bis 1,2 Gew.-Teile Phosphor als $P_2O_5$, 0 bis 5 Gew.-Teile Antimon als $Sb_2O_5$ und 0,05 bis 2 Gew.-Teile Silber als $Ag_2O$, wobei der Gesamtgehalt an Niob, Phosphor und Antimon nicht Null ist.

3. Katalysator nach Anspruch 1 oder 2, dadurch gekennzeichnet, daß in der Schicht aus katalytisch aktiver Substanz, die sich auf dem hitzebeständigen anorganischen Träger befindet, das Porenvolumen, das aus Poren mit einem Durchmesser von 0,15 bis 0,45$\mu$m besteht, 50% oder mehr ausmacht als das gesamte feine Porenvolumen, das aus feinen Poren mit einem Durchmesser von 10 $\mu$m oder weniger besteht.

**Revendications**

1. Catalyseur pour la préparation de l'anhydride phtalique par oxydation catalytique en phase vapeur d'o-xylène et/ou de naphtalène avec de l'oxygène moléculaire ou un gaz contenant de l'oxygène moléculaire, ayant une substance catalytique activée supportée sur un support minéral résistant à la chaleur, laquelle substance comprend :

   (A) 1 à 20 parties en poids d'oxyde de vanadium sous la forme $V_2O_5$ et 99 à 80 parties en poids de dioxyde de titane du type anatase ayant une surface spécifique de 10 à 60 $m^2/g$, sous la forme $TiO_2$, et, pour 100 parties en poids total de (A),
   (B) 0,05 à 1,2 partie en poids d'au moins un élément choisi parmi le potassium, le césium, le rubidium et le thallium, sous la forme de leurs oxydes, et 0,05 à 2 parties en poids d'argent sous la forme $Ag_2O$.

2. Catalyseur pour la préparation de l'anhydride phtalique par oxydation catalytique en phase vapeur d'o-xylène et/ou de naphtalène avec de l'oxygène moléculaire ou un gaz contenant de l'oxygène moléculaire, ayant une substance catalytique active supportée sur un support minéral résistant à la chaleur, laquelle substance comprend :

(A) 1 à 20 parties en poids d'oxyde de vanadium sous la forme $V_2O_5$ et 99 à 80 parties en poids de dioxyde de titane du type anatase ayant une surface spécifique de 10 à 60 m²/g, sous la forme $TiO_2$, et, pour 100 parties en poids de (A),

(C) 0 à 1 partie en poids de niobium sous la forme $Nb_2O_5$, 0,05 à 1,2 partie en poids d'au moins un élément choisi parmi le potassium, le césium, le rubidium et le thallium, sous la forme de leurs oxydes, 0 à 1,2 partie en poids de phosphore sous la forme $P_2O_5$, 0 à 5 parties en poids d'antimoine sous la forme $Sb_2O_3$, et 0,05 à 2 parties en poids d'argent sous la forme $Ag_2O$, la quantité totale de niobium, de phosphore et d'antimoine ne valant pas zéro.

3.  Catalyseur selon la revendication 1 ou 2, où, dans la couche de substance catalytique active supportée sur le support minéral résistant à la chaleur, le volume occupé par des pores ayant un diamètre de 0,15 à 0,45 µm représente 50 % ou plus du volume total occupé par des pores fins ayant un diamètre de 10 µm ou moins.